# NOUVEAU FASCICULE DE BREVET EUROPEEN

(11) **EP 0 680 751 B2**
(45) Date de publication et mention de la décision concernant l'opposition: **24.11.2004**
(45) Mention de la délivrance du brevet: 23.08.2000
(21) Numéro de dépôt: 95400967.6
(22) Date de dépôt: 27.04.1995
(51) Int. Cl.: A61K 7/48, A61K 7/00, A23D 9/00, A23L 1/22

(54) **Utilisation cosmétique d'émulsions aqueuses contenant des vésicules lipidiques**
Kosmetische Verwendung wässeriger Emulsionen welche Lipidvesikel enthalten
Cosmetical use of aqueous emulsions containing lipid vesicles

(30) Priorité: 02.05.1994 FR 9405319
(43) Date de publication de la demande: 08.11.1995
(73) Titulaire: LABORATOIRES DE BIOLOGIE VEGETALE YVES ROCHER, 56200 La Gacilly (FR)
(72) Inventeur: Voultoury, Robert, F-92160 Antony (FR); Flavigny, Philippe, F-91790 Boissy sous Saint Yon (FR)
(74) Mandataire: Bernasconi, Jean Raymond

(56) Documents cités:
- EP-A- 0 302 769
- WO-A-91/11169
- LU-A- 86 021
- US-A- 4 088 795
- US-A- 4 324 802
- US-A- 4 437 895
- US-A- 4 701 471
- US-A- 4 810 498
- US-A- 4 892 727
- J. OF CELL BIOLOGY, vol.117, no.2, Avril 1992, US pages 327 - 335 TZEN ET AL. 'SURFACE STRUCTURE AND PROPERTIES OF PLANT SEED OIL BODIES'
- PARFUMS, COSMETIQUES, AROMES, vol.56, Mai 1984, FR page 57 RAYMOND ET AL. 'LES PROTEINES D'OLEAGINEUX: PROPRIETES FONCTIONNELLES UTILISABLES EN COSMETOLOGIE'
- J.T.C Tzen et al.,J.Cell Biol.,117(2),327-335(1992)
- Yatsu et al, Plant Physiol., 48, 675-682 (1971)
- Oxford Dict. of Biochem. and Mol. Biol., Oxford University Press, 681 (1971)
- http://www.nf-2000.org/secure/Crops/S590.ht m
- F.B. Salisbury et al, Plant Physiol., 308-311 (1991)
- Henderson's Dict. of Biol. Terms, 358 (1989)
- http:// www.bartleby.com/61/21/O0062100.html
- Dict. d'agricult. et des sciences annexes, Agence de Coop. Cult. et Techn., page 168 (1977)
- L. Tiaz et al, Plant Physiology, 2nd ed., Sinauer Ass. Inc., 25-26 and 312-313
- Dict. d'agricult. et des sciences annexes, Agence de Coop. Cult. et Techn., 118 (1977)
- http://www.prolea.com/gauche.htm
- K.Faupel et al, NCAT Agriculture Specialists, 1-8 (2002)
- Oxford Dict. of Biochem. and Mol. Biol., Oxford University Press, 370 and 499 (1997)
- D.H.Putnam, An Interdisciplinary Approach to the Development of Lupin as an Alternative Crop, 266-277 (1993)

## Description

La présente invention concerne l'utilisation cosmétique d'émulsions aqueuses contenant des vésicules lipidiques et un procédé pour la fabrication de ces émulsions.

La présente invention concerne plus spécifiquement l'utilisation cosmétique d'émulsions comprenant des vésicules lipidiques qui comprennent comme enveloppe les protéines et les phospholipides présents dans les graines d'oléagineux.

On a considéré jusqu'à présent que dans les graines d'oléagineux, l'huile (triglycérides essentiellement) était présente sous la forme de corps huileux d'un diamètre moyen de 1 à 10 micromètres comprenant un noyau central d'huile entouré d'une enveloppe constituée de protéines (oléosines) et de phospholipides (J. Tzen et al., J. Cell Bio, 117, 327, 1992).

Par ailleurs, J. Raymond et al. (Parfums, cosmétiques, arômes, vol. 56, mai 1984) ont décrit la séparation d'un isolat protéique à partir de tourteaux de tournesol pour une utilisation en cosmétologie.

De plus, WO 91/11169 a décrit des compositions cosmétiques contenant un extrait de tourteaux de tournesol comprenant essentiellement des polyphénols.

On a maintenant découvert que les protéines constituant l'enveloppe des corps huileux sont de deux types : certaines protéines ne sont pas glycosylées et d'autres protéines sont glycosylées.

La présente invention décrit l'utilisation cosmétique d'émulsions comprenant des vésicules lipidiques qui comprennent l'ensemble des protéines et des phospholipides présents dans l'enveloppe des corps huileux et donc qui comprennent à la fois des protéines non glycosytées et des protéines glycosylées identiques à celles présentes dans l'enveloppe des corps huileux.

Par ailleurs, on sait que lors de l'extraction des huiles végétales des oléagineux, on récupère des tourteaux qui contiennent encore une fraction importante des protéines et des phospholipides présents dans l'enveloppe des corps huileux. Ces tourteaux contiennent en outre un taux d'huile non extraite qui peut représenter de 10 à 30 % en poids.

A cet effet, la présente invention a pour objet l'utilisation cosmétique d'emulsions comprenant des vésicules lipidiques ayant une taille moyenne de 0,1 à 20 micromètres, notamment 1 à 8 micromètres, et comprenant une enveloppe constituée pratiquement de l'ensemble des protéines et des phospholipides présents dans les corps huileux des graines d'oléagineux, entourant un noyau comprenant au moins des lipides exogènes et des substances lipophiles exogènes, ladite émulsion étant obtenue par le procédé décrit ci-après, en valorisant les tourteaux d'oléagineux.

L'invention a plus précisément pour objet l'utilisation cosmétique de telles compositions comprenant une émulsion aqueuse comprenant des vésicules lipidiques dispersées dans une phase aqueuse.

Dans la présente invention l'expression "pratiquement l'ensemble des protéines et des phospholipides présent dans les corps huileux des graines d'oléagineux" signifie que l'on retrouve dans l'enveloppe des vésicules pratiquement les mêmes constituants que dans l'enveloppe des corps huileux et que les protéines comprennent à la fois des protéines non glycosylées et des protéines glycosylées. En pratique, les protéines et les phospholipides présents dans les graines d'oléagineux sont ceux présents dans les tourteaux d'oléagineux.

L'invention a également pour objet un procédé de fabrication d'une telle émulsion aqueuse, qui comprend :
- le broyage de tourteaux d'oléagineux,
- l'addition aux tourteaux broyés d'une phase lipidique de façon à avoir un pourcentage global de lipides de 50 à 95 % en poids,
- le malaxage des tourteaux broyés et de la phase lipidique jusqu'à obtenir une pâte homogène, notamment à une température de 0 à 90° C,
- l'addition d'une phase aqueuse à la pâte dans un rapport pondéral pâte/phase aqueuse d'environ 40/60 à 5/95, notamment à une température de 0 à 90° C,
- l'agitation de la pâte et de la phase aqueuse pour former une émulsion,
- la décantation et/ou la filtration de l'émulsion pour éliminer les particules solides,
- et/ou la centrifugation de l'émulsion pour obtenir une émulsion concentrée.

Comme exemples de tourteaux d'oléagineux, on peut citer les tourteaux de soja, pistache, macadamia, tournesol, colza, arachide, amande, noisette, sésame, bourrache, germe de blé, jojoba. On utilise de préférence des tourteaux obtenus à partir de graines à forte teneur en huile (pistache, macadamia, arachide, jojoba, noisette, amande).

Le broyage des tourteaux peut être effectué par des broyeurs classiques, tels que des broyeurs à couteaux, avantageusement jusqu'à obtenir une taille de particules inférieure à 0,1 mm.

Les tourteaux broyés peuvent être éventuellement soumis à une irradiation pour une débactérisation jusqu'à une intensité de 10 kgray et conservés sous atmosphère inerte en emballage hermétiquement clos.

Par lipides exogènes on désigne les lipides d'apport, c'est-à-dire que l'on ajoute et qui ne sont pas présents dans les tourteaux broyés. Ces lipides que l'on ajoute aux tourteaux broyés peuvent être des lipides du même type que ceux présents dans l'oléagineux utilisé (lipides endogènes) ou d'autres lipides. En général, il reste dans les tourteaux une proportion relativement importante de triglycérides et l'on ajoute des lipides de façon à avoir un pourcentage global de lipides de 50 à 95 % en poids.

On règle la quantité de lipides en fonction de la taille des particules de l'émulsion finale que l'on veut obtenir. Ainsi, si l'on veut avoir des tailles de particules plus faibles, on diminue la quantité de lipides ajoutés.

Outre ces lipides, on ajoute des substances lipophiles telles que des vitamines (vitamines D, A, E, K), des filtres solaires (tels que Parsol MCX et Parsol 1789 de Givaudan ou de la benzophénone-3), des mélanges lipophiles complexes (Titan M262CD de Kemira Oy) additionnés éventuellement d'huile minérale, de carotène, de silicone (par exemple DC 200 de Dow Corning), d'esters gras.

Le malaxage des tourteaux et de la phase lipidique ajoutée est alors effectué jusqu'à obtenir une pâte homogène. On opère avantageusement sous atmosphère non oxydante (sous vide ou sous azote).

Les vésicules lipidiques peuvent contenir de 0,01% à 100% en poids de lipides exogènes ou de substances lipophiles exogènes.

La phase aqueuse est ensuite ajoutée, avantageusement à température ambiante et sous atmosphère inerte (sous vide ou sous azote). Cette phase aqueuse peut comprendre, outre de l'eau, différents constituants hydrophiles.

L'addition de la phase aqueuse est généralement réalisée de façon à avoir un rapport pondéral pâte/phase aqueuse d'environ 40/60 à 5/95. On préfère un rapport 30/70 à 5/95.

Pour obtenir une émulsion, on soumet l'ensemble à une agitation qui peut être importante, à l'aide par exemple d'un broyeur colloïdal.

L'émulsion obtenue est alors soumise éventuellement à une décantation et/ou filtration, par exemple sur tamis de 200 micromètres.

L'émulsion obtenue est relativement diluée et en général il est nécessaire de concentrer cette émulsion par centrifugation. A cet effet, on peut utiliser une centrifugeuse à assiettes adaptée au taux de la phase lipidique. Le produit obtenu est un concentrat de particules lipidiques dont le taux de composants lipophiles peut varier d'environ 20 à 70 % en poids suivant les conditions de fabrication.

Avant ou après cette centrifugation, la suspension lipidique peut subir un traitement thermique dont les conditions peuvent varier de 2 secondes à 10 minutes pour des températures de 80° C à 140° C. On peut alors procéder, si désiré, à une opération d'homogénéisation à l'aide d'un appareil de type Gaulin par exemple, laquelle opération peut avoir des conditions de pression variant de 5 à 400.10⁵ Pa.

Ce concentrat de particules lipidiques peut être repris et dilué avec une phase aqueuse du type décrit plus loin. Cette phase aqueuse peut être épaissie à l'aide de gélifiants tels que gomme xanthane, gomme sclerane, bentone et dérivés, cellulose et dérivés, carbopol et dérivés, caroube, carraghénanes et dérivés, présents à des concentrations de 0 à 2 % en poids.

Le concentrat de particules lipidiques peut en outre être baratté suivant les techniques de laiteries pour obtenir un beurre que l'on comprime pour en exprimer l'eau.

Les émulsions ainsi obtenues ont en général des tailles moyennes de particules de 0,1 à 20 micromètres.

Les émulsions ainsi obtenues trouvent notamment des applications dans le domaine des produits cosmétiques et la présente invention a également pour objet l'utilisation cosmétique des compositions comprenant des vésicules lipidiques selon l'invention (par exemple des compositions hydratantes, des compositions de protection solaire, des compositions nourrissantes).

L'utilisation dans ces compositions d'un système émulsionnant naturel (oléosines, phospholipides) entraîne une meilleure tolérance cutanée du produit final.

Dans ce cadre de la préparation d'une émulsion à usage cosmétique, on peut par exemple utiliser une phase aqueuse comprenant:
- Glycérine 5 à 10%
- Propylène glycol 5 à 10 %
- Butane diol 5 à 10 %
- Urée 1 à 5%
- Sodium PCA 0,5 à 5 %
- EDTA 0,05 à 0,1 %
- Méthyl paraben 0,05 à 0,2 %
- Propyl paraben 0,05 à 0,1 %
- Butyl paraben 0,05 à 0,1 %
- Ethanol 0,05 à 0,5 %
- BHT 0,05 à 1%
- Alginate de sodium 1 à 5 %
- Vitamine C et dérivés 0,05 à 1 %
- Vitamine B et dérivés 0 à 1 %
- Acide sorbique 0 à 1 %
- Préparations aqueuses diverses d'oligo-éléments
- Sulfite de sodium 0 à 0,4 %.

En outre, il est à noter que les émulsions obtenues comprennent des protéines qui présentent une grande similitude avec les apolipoprotéines des mammifères; autrement dit, on pense que les lipases des mammifères reconnaissent les protéines des émulsions selon l'invention, ce qui permet la fixation des lipases sur les membranes des particules et par suite la dégradation des lipides substrats des lipases concernées.

Les exemples suivants illustrent la présente invention.

### EXEMPLE 1

On broie un tourteau d'arachide à l'aide d'un broyeur à couteaux jusqu'à obtenir une taille inférieure à 0,1 mm environ.

Le tourteau contient 20 % en poids environ de triglycérides.

On ajoute à 50 parties de tourteau broyé 50 parties d'huile végétale (triglycérides d'arachide ou autre oléagineux). L'ensemble est malaxé sous vide à température ambiante jusqu'à obtenir une pâte homogène.

On ajoute à cette pâte 900 parties d'eau. On soumet l'ensemble à température ambiante et sous vide à une vive agitation à l'aide d'un broyeur colloïdal réglé sur l'ouverture maximale.

On filtre sous tamis de 200 micromètres. On centrifuge éventuellement le filtrat qui est un lait très fluide dans une centrifugeuse de laiterie.

On obtient un concentrat de particules lipidiques ayant une taille moyenne de 3 micromètres, contenant environ 60 % de triglycérides.

### EXEMPLE 2

On opère comme à l'exemple 1 avec un tourteau de macadamia (contenant 25 % en poids de triglycérides), en ajoutant à 30 parties en poids de tourteaux, 70 parties en poids de triglycérides (huile de macadamia ou d'autres oléagineux).

L'eau est ajoutée à la pâte à raison de 900 parties en poids.

On obtient finalement un concentrat de particules lipidiques ayant une taille moyenne de 3 micromètres, ce concentrat contenant environ 60 % de triglycérides.

On donnera ci-après des exemples de compositions obtenues selon le procédé de l'invention.

### Exemple A - Lait pour le visage

. Eau qs. 100 %
. Gomme xanthane 1 à 2 %
. Concentrat A 20 à 40 %
. Parfum 0,1 à 0,5 %
. Conservateurs qs.

Le concentrat A est fabriqué comme décrit à l'exemple 1, à partir d'une huile végétale.

Le pH varie de 5,5 à 6,5 (qs = acide citrique).
Viscosité de 2000 à 3000 cP.

### Exemple B - Lait solaire écran total

. Eau qs 100 %
. Gomme xanthane 1 à 2 %
. Concentrat B 20 à 40 %
. Parfum 0,1 à 0,5 %
. Conservateurs qs.

Le concentrat B est fabriqué comme décrit à l'exemple 1, à partir d'une huile composée par exemple de :
. Triglycérides qs 100%
. PARSOL 1789 1 à 5 %
. PARSOL MCX 1 à 15 %
. TITAN M262CD 1 à 10 %

Le pH varie de 5,5 à 6,5 (qs = acide citrique).
Viscosité 2000 à 3000 cP.

### Exemple C - Lait hydratant pour le corps

. Eau qs 100 %
. Glycérine 5 à 10 %
. Gomme xanthane 1 à 2 %
. Urée 1 à 5 %
. Concentrat C 20 à 40%
. Parfum 0,1 à 0,5 %
. Conservateurs qs

Le concentrat C est fabriqué comme décrit à l'exemple 1, à partir d'huile végétale additionnée d'esters gras du jojoba (5 à 10 %).

Le pH varie de 6,0 à 6,5 (qs = acide citrique), la viscosité de 2000 à 3000 cP.

On donnera ci-après des résultats d'essais mettant en évidence les natures différentes des protéines présentes dans l'enveloppe des corps huileux.

### EXEMPLE 3

On a procédé comme à l'exemple 2 en partant d'un tourteau de macadamia.

A partir d'un concentrat de vésicules lipidiques, on a séparé les protéines par la méthode sur gradients d'urée après extraction des lipides à l'éther diéthylique (Millichip, Colloque Int. Lipides Végétaux).

Les protéines ont été remises en solution dans un tampon Tris/HCl 10 mM, SDS 0,07 M, pH 8.

La purification des protéines a été réalisée par chromatographie de tamisage moléculaire sur colonne de Superose 6 équilibrée et éluée en tampon Tris/HCI 10 mM SDS 0,07 M, pH 8,2.

On a séparé 50 fractions et testé les fractions 7, 36 et 43 correspondant à des pics d'élution. A cet effet on a utilisé une technique de type "dot-blot" avec un sérum dirigé contre la β-glucosidase du soja qui réagit avec les motifs glucidiques complexes communs à la plupart des plantes, qui contiennent des résidus α-1-3 glucose et β-1-2-xylose.

Les fractions 7 et 36 sont reconnues par le sérum dirigé contre la β-glucosidase, ce qui n'est pas le cas de la fraction 43. La reconnaissance est donc imputable à la présence de chaînes glycosidiques dans les fractions 7 et 36 qui n'apparaissent pas pour la fraction 43.

## Revendications

1. Utilisation cosmétique d'une émulsion comprenant dans une phase aqueuse des vésicules lipidiques ayant une taille moyenne de 0,1 à 20 micromètres et comprenant une enveloppe constituée pratiquement de l'ensemble des protéines et des phospholipides présents dans les corps huileux des graines d'oléagineux, entourant un noyau comprenant des lipides exogènes et des substances lipophiles exogènes,
ladite émulsion obtenue par :
(a) le broyage due tourteaux d'oléagineux,
(b) l'addition aux tourteaux broyés d'une phase lipidique comprenant lesdits lipides exogènes et lesdites substances lipophiles exogènes, de façon à avoir un pourcentage global de lipides de 50 à 35% en poids;
(c) le malaxage des tourteaux broyés et de la phase lipidique jusqu'à obtenir une pâte homogène ;
(d) l'addition d'une phase aqueuse à la pâte dans un rapport pondéral pâte/phase aqueuse d'environ 40/60 à 5/95;
(e) l'agitation de la pâte et de la phase aqueuse pour former une émulsion ; et
(f) la décantation et/ou la filtration de l'émulsion ;
et/ou (g) la centrifugation de l'émulsion pour obtenir une émulsion concentrée.

2. Procédé de fabrication d'une émulsion aqueuse destinée à une utilisation selon la revendication 1, ce procédé comprenant :
(a) le broyage de tourteaux d'oléagineux;
(b) l'addition aux tourteaux broyés d'une phase lipidique, de façon à avoir un pourcentage global de lipides de 50 à 95% en poids ;
(c) le malaxage des tourteaux broyés et de la phase lipidique jusqu'à obtenir une pâte homogène ;
(d) l'addition d'une phase aqueuse à la pâte dans un rapport pondéral pâte/phase aqueuse d'environ 40/60 à 5/95 ;
(e) l'agitation de la pâte et de la phase aqueuse pour former une émulsion ; et
(f) la décantation et/ou la filtration de l'émulsion ;
et/ou (g) lacentrifugation de l'émulsion pour obtenir une émulsion concentrée.

3. Procédé selon la revendication 2, où l'émulsion est centrifugée selon l'étape (g), et où on effectue en outre un barattage des l'émulsion ainsi concentrée.

4. Procédé selon l'une des revendications 2 à 3, où on effectue l'addition de la phase aqueuse et son mélange avec la pâte à température ambiante.

5. Procédé selon l'une des revendications 2 à 4, où on effectue l'addition de la phase aqueuse et son mélange avec la pâte sous atmosphère non oxydante.

## Patentansprüche

1. Kosmetische Verwendung einer Emulsion, die in einer wäßrigen Phase Lipidvesikel einer mittleren Größe von 0,1 bis 20 µm enthält und eine Hülle enthält, die praktisch durch die Gesamtheit der Proteine und Phospholipide, die im Inneren des öligen Körpers der ölhaltigen Körner vorliegen, gebildet ist, umgebend einen exogene Lipide und lipophile exogene Substanzen enthaltenden Kern, wobei diese Emulsion erhalten wird durch:
a) das Reiben des ölhaltigen Kuchens,
b) die Zugabe einer lipidartigen Phase, enthaltend diese exogenen Lipide und diese exogenen lipophilen Substanzen, zu dem geriebenen Kuchen in der Weise, dass der Gesamtanteil der Lipide 50 bis 95 Gew.% beträgt,
c) das Kneten des geriebenen Kuchens und der lipidartigen Phase bis zum Erhalt einer homogenen Paste,
d) die Zugabe einer wäßrigen Phase zu der Paste in einem Gewichtsverhältnis Paste/wäßrige Phase von etwa 40/60 bis 5/95,
e) Bewegen der Paste und der wäßrigen Phase zur Bildung einer Emulsion, und
f) Dekantieren und/oder Filtrieren der Emulsion, und/oder
g) Zentrifugieren der Emulsion, um eine konzentrierte Emulsion zu erhalten.

2. Verfahren zur Herstellung einer wäßrigen Emulsion, die für eine Verwendung gemäß Anspruch 1 bestimmt ist,
a) das Reiben des ölhaltigen Kuchens,
b) die Zugabe einer lipidartigen Phase zu dem geriebenen Kuchen in der Weise, dass der Gesamtanteil der Lipide 50 bis 95 Gew.% beträgt,
c) das Kneten des geriebenen Kuchens und der lipidartigen Phase bis zum Erhalt einer homogenen Paste,
d) die Zugabe einer wäßrigen Phase zu der Paste in einem Gewichtsverhältnis Paste/wäßrige Phase von etwa 40/60 bis 5/95,
e) Bewegen der Paste und der wäßrigen Phase zur Bildung einer Emulsion, und
f) Dekantieren und/oder Filtrieren der Emulsion, und/oder
g) Zentrifugieren der Emulsion, um eine konzentrierte Emulsion zu erhalten.

3. Verfahren nach Anspruch 2, in dem man die Emulsion gemäß Verfahrensschritt (g) zentrifugiert, und/oder in dem man ferner eine Butterung der konzentrierten Emulsion bewirkt.

4. Verfahren nach einem der Ansprüche 2 bis 3, in dem man die Zugabe der wäßrigen Phase und deren Vermischen mit der Paste bei Raumtemperatur durchführt.

5. Verfahren nach einem der Ansprüche 2 bis 4, in dem man die Zugabe der wäßrigen Phase und sein Vermischen mit der Paste unter einer nicht oxidierenden Atmosphäre bewirkt.

## Claims

1. Use as a cosmetic of an emulsion comprising lipidic vesicles in an aqueous phase, having a mean size of 0.1 to 20 micrometers and comprising an envelope formed essentially of the set of proteins and phospholipids present in the oily bodies of oleaginous seeds, surrounding a core comprising exogenous lipids and exogenous lipophilic substances, the said emulsion being obtained by:
(a) grinding oil cakes;
(b) adding a lipidic phase comprising the said exogenous lipids and the said exogenous lipophilic substances to the ground cakes, in such a manner as to obtain a total percentage of lipids from 50 to 95% by weight;
(c) kneading the ground cakes and the lipidic phase until a homogenous paste is obtained;
(d) adding an aqueous phase to the paste in a ratio by weight of paste to aqueous phase of around 40/60 to 5/95;
(e) agitating the paste and the aqueous phase to form an emulsion; and
(f) decanting and/or filtering the emulsion;
and/or (g) centrifuging the emulsion to obtain a concentrated emulsion.

2. A method of making an aqueous emulsion-for a use according to claim 1, the method comprising:
(a) grinding oil cakes;
(b) adding a lipidic phase to the ground cakes, in such a manner as to obtain a total percentage of lipids from 50 to 95% by weight;
(c) kneading the ground cakes and the lipidic phase until a homogenous paste is obtained;
(d) adding an aqueous phase to the paste in a ratio by weight of paste to aqueous phase of around 40/60 to 5/95;
(e) agitating the paste and the aqueous phase to form an emulsion: and
(f) decanting and/or filtering the emulsion,
and/or centrifuging the emulsion to obtain a concentrated emulsion.

3. A method according to claim 2, in which the emulsion is centrifuged according to step (g) and in which the emulsion thus concentrated is further churned.

4. A method according to either of claims 2 and 3, in which the addition of the aqueous phase and its mixing with the paste are effected at ambient temperature.

5. A method according to any of claims 2 to 4, in which the addition of the aqueous phase and its mixing with the paste are effected under a non-oxidising atmosphere.
